# EUROPÄISCHE PATENTSCHRIFT

(11) **EP 3 394 441 B1**
(45) Veröffentlichungstag und Bekanntmachung des Hinweises auf die Patenterteilung: **09.10.2019**
(21) Anmeldenummer: 16819266.4
(22) Anmeldetag: 20.12.2016
(51) Int. Cl.: A61M 1/36

(54) **BLUTBEHANDLUNGSGERÄT UMFASSEND EINE DOSIERLEITUNG MIT EINER MEMBRANPUMPE UND EINEM VENTIL UND VERFAHREN ZUR DOSIERUNG**
BLOOD TREATMENT DEVICE COMPRISING A DOSING LINE HAVING A DIAPHRAGM PUMP AND HAVING A VALVE AND METHOD FOR DOSING
APPAREIL DE TRAITEMENT DU SANG COMPRENANT UNE CONDUITE DE DOSAGE AVEC UNE POMPE À MEMBRANE ET UNE VANNE, ET PROCÉDÉ DE DOSAGE

(30) Priorität: 21.12.2015 DE 102015016670
(43) Veröffentlichungstag der Anmeldung: 31.10.2018
(73) Patentinhaber: Fresenius Medical Care Deutschland GmbH, 61352 Bad Homburg (DE)
(72) Erfinder: PETERS, Arne, 61352 Bad Homburg (DE)
(74) Vertreter: Herrmann, Uwe
(86) Internationale Anmeldenummer: PCT/EP2016/002140
(87) Internationale Veröffentlichungsnummer: WO 2017/108176

(56) Entgegenhaltungen:
- EP-A1- 1 746 479
- DE-A1- 4 118 600
- DE-C1- 4 228 193
- US-B1- 6 604 908

## Beschreibung

Die Erfindung betrifft ein Blutbehandlungsgerät umfassend eine Dosierleitung mit einer Membranpumpe und einem Sperr- und Drosselventil.

Bei der Dialyse ist die richtige Dosierung von Flüssigkeiten von zentraler Bedeutung. Große Flüssigkeitsströme wie der Dialysierflüssigkeitsstrom werden oft über Bilanziersysteme gefördert. Mittlere Flüssigkeitsströme wie der extrakorporale Blutstrom werden oft durch Schlauchrollenpumpen gefördert. Flussfehler von bis zu etwa 10% werden dabei in Kauf genommen.

Eine höhere Anforderung an die Dosiergenauigkeit besteht aber bei der Zudosierung kleiner Mengen einer Wirkstofflösung, beispielsweise bei der Zudosierung von einer Antikoagulationsmittellösung in den extrakorporalen Blutkreislauf. Hierbei soll der Flussfehler in jedem Fall unter 5% liegen. Im Stand der Technik werden dafür oftmals Schlauchrollenpumpen oder Spritzenpumpen eingesetzt. Der Einsatz von Membranpumpen ist hingegen für derartige Anwendungen im Stand der Technik kaum verbreitet, da unerwünschte Flusspulse erzeugt werden.

Die Flusspulse sind darauf zurückzuführen, dass in Membranpumpen die Membran durch ihre Elastizität, durch ihre Verformung und durch ihre Vorspannung das im Idealfall zwischen den beiden Kammern der Membranpumpe herrschende Druckgleichgewicht beeinflusst. Für eine pneumatische Membranpumpe gilt im statischen Fall der Zusammenhang P_{PNEU} - P_{HYD} = P_{MEM}. Der Membrandruck P_{MEM} ist nicht konstant und stellt daher bei der Regelung der Pumpe einen Störfaktor dar, da sich der Flüssigkeitsdruck P_{HYD} nicht proportional mit dem Regelungsdruck P_{PNEU} verändert. Der Einfluss dieses Störfaktors ist relativ gesehen groß, wenn Flüssigkeitsdruck P_{HYD} und Regelungsdruck P_{PNEU} gering sind. Dies ist beispielsweise dann der Fall, wenn anhand der Membranpumpe geringe Flussraten erzeugt werden sollen. Ein gleichartiges Blutbehandlungsgerät ist bspw. aus der US 6 604 908 bekannt.

Vor allem bei der Gabe von Citrat als Antikoagulationsmittel muss aber ein konstanter Fluss aufrechterhalten werden.

Aufgabe der Erfindung, wie in Anspruch 1 definiert, ist es, den Einfluss dieses Störfaktors auch bei geringen Flussraten zu reduzieren.

Vor diesem Hintergrund betrifft die Erfindung ein Blutbehandlungsgerät mit wenigstens einer Dosierleitung, die in einen Flüssigkeitskreislauf mündet, wobei in der Dosierleitung ein Fördermodul umfassend eine Membranpumpe und ein druckseitig davon angeordnetes Ventil angeordnet sind. Das Ventil ist so ausgebildet, dass es sowohl als Sperrventil als auch als Drosselventil wirken kann. Die der Erfindung zugrundeliegende Idee besteht darin, durch eine Verringerung des Strömungsquerschnitts stromabwärts der Pumpe den Flusswiderstand zu vergrößern und damit einen Druckoffset für den Flüssigkeitsdruck P_{HYD} (und folglich den Regelungsdruck P_{PNEU}) zu erzeugen, der den Einfluss des gleichbleibenden Membrandrucks P_{MEM} verringert. Dadurch kann der Fluss der geförderten Flüssigkeit geglättet werden und es lassen sich auch geringe Flüsse regeln. Weitere Vorteile bestehen darin, dass für die Konstruktion der Pumpenmembran weniger elastische und daher ggf. kostengünstigere Kunststoffe eingesetzt werden können und dass die Geometrie der Membran weniger entscheidend ist.

In einer Ausführungsform handelt es sich bei dem Blutbehandlungsgerät um ein Dialysegerät.

In einer Ausführungsform handelt es sich bei der Dosierleitung um eine Dosierleitung für eine Lösung eines koagulationshemmenden Mittels, die in einen extrakorporalen Blutkreislauf des Blutbehandlungsgeräts mündet.

In einer Ausführungsform ist die Membranpumpe eine pneumatisch betriebene Membranpumpe. In einer Ausführungsform ist das Ventil ein pneumatisch betriebenes Ventil. Sofern sowohl die Pumpe als auch das Ventil pneumatisch betrieben werden, kann vorgesehen sein, dass die beiden Aktoren an ein gemeinsames Pneumatikmodul angeschlossen sind. Vorzugsweise ist das Ventil so ausgebildet, dass sein Schließzustand proportional dem Pneumatikdruck ist.

In einer Ausführungsform sind die Membranpumpe und das Ventil in einem gemeinsamen Gehäuse des Fördermoduls angeordnet. Es kann vorgesehen sein, dass das Gehäuse wenigstens zwei lösbar aneinander befestigte Teile umfasst, wobei ein erster Teil die Pumpenmechanik und die Ventilmechanik umfasst und ein zweiter Teil wenigstens einen Abschnitt der flüssigkeitsführenden Dosierleitung definiert. Bei dem ersten Teil kann es sich um ein Reusable handeln und bei dem zweiten Teil um ein lösbar in das Reusable eingelegtes Disposable.

Nach der Idee der Erfindung weist das Blutbehandlungsgerät eine Steuereinheit auf, welche die Pumpe und das Ventil ansteuert, vorzugsweise mittels des Pneumatikmoduls. Dabei ist auf der Steuereinheit ein Algorithmus hinterlegt, der so ausgebildet ist, dass im Ventil während einer Druckphase der Pumpe der Strömungsquerschnitt gegenüber dem maximalen Strömungsquerschnitt verkleinert wird, um den Flusswiderstand zu vergrößern und so einen Gegendruck in der Flüssigkeitskammer der Pumpe zu erzeugen. Der Strömungsquerschnitt kann beispielsweise um die Hälfte oder mehr als die Hälfte verkleinert werden. So kann erreicht werden, dass der Druckoffset signifikant höher ist als der störende Membrandruck P_{MEM}, beispielsweise mindestens dreimal höher.

In einer Ausführungsform ist der in der Steuereinheit hinterlegte Algorithmus ferner so ausgebildet, dass während der Druckphase der Pumpe der reduzierte Strömungsquerschnitt im Ventil fortlaufend vergrößert wird, um bei einem abnehmenden Flüssigkeitsdruck in der Pumpe eine konstante Strömungsrate aus dem Fördermodul zu gewährleisten. Dabei kann ferner vorgesehen sein, dass während der Druckphase der Pumpe das pneumatische Volumen in der Regelungskammer der Pumpe konstant gehalten wird. In dieser Ausgestaltung ändert sich der Regelungsdruck P_{PNEU} nur aufgrund der aus der Pumpenkammer verdrängten Flüssigkeit. Die Flussrate der Flüssigkeit kann präzise anhand des Öffnungszustandes des Ventils geregelt werden.

In einer Ausführungsform ist der in der Steuereinheit hinterlegte Algorithmus ferner so ausgebildet, dass der reduzierte Strömungsquerschnitt im Ventil und der Flüssigkeitsdruck in der Pumpe während der Druckphase der Pumpe konstant gehalten werden, um eine konstante Strömungsrate aus dem Fördermodul zu gewährleisten. In dieser Ausführungsform wird beispielsweise das pneumatische Volumen hinter der Membran aktiv geregelt, um den Regelungsdruck P_{PNEU} bei einem veränderten Volumen auf der Flüssigkeitsseite konstant zu halten und die Flussrate der Flüssigkeit bei gleichbleibendem Öffnungszustandes des Ventils präzise anhand des Regelungsdrucks regeln zu können.

Um für die Regelung geeignete Informationen über den Flüssigkeitsfluss oder die Drücke zu erhalten, können sowohl im Flüssigkeitssystem als auch im Pneumatiksystem Fluss- und/oder Drucksensoren angeordnet sein, die mit der Steuereinheit in Verbindung stehen.

In einer Ausführungsform umfasst das Fördermodul ferner ein saugseitiges Sperrventil, welches vorzugsweise im selben Gehäuse wie die Membran und das druckseitige Ventil angeordnet ist und/oder am selben Pneumatikmodul angeschlossen ist. Das saugseitige Sperrventil kann identisch ausgeführt sein wie das druckseitige Drosselventil. Es kann durch die Steuereinheit vorzugsweise mittels des Pneumatikmoduls so ansteuert werden, dass es während der Druckphase der Pumpe geschlossen ist.

Die vorliegende Erfindung betrifft des Weiteren ein Verfahren zur Dosierung eines Mediums, wie z.B. eines Wirkstoffes, das vorzugsweise in einem Blutbehandlungsgerät gemäß einem der Ansprüche durchgeführt wird. Das Verfahren betrifft vorzugsweise die Zudosierung wenigstens eines Wirkstoffes, wie beispielsweise eines Antikoagulationsmittels in einen Flüssigkeitsstrom oder in eine Flüssigkeit, bei der es sich z. B. um eine Dialysierflüssigkeit oder eine Flüssigkeit handelt, aus der Dialysierflüssigkeit hergestellt wird. Das Verfahren ist dadurch gekennzeichnet, dass zur Dosierung ein in einer Dosierleitung befindliches Fördermodul eingesetzt wird, das eine Membranpumpe und ein druckseitig davon angeordnetes Ventil umfasst, das sowohl als Sperrventil als auch als Drosselventil wirken kann.

Weitere vorteilhafte Ausgestaltungen des Verfahrens sind Gegenstand der Unteransprüche.

Denkbar ist es, dass die Pumpe und das Ventil vorzugsweise mittels eines Pneumatikmoduls so ansteuert werden, dass im Ventil während einer Druckphase der Pumpe der Strömungsquerschnitt gegenüber dem maximalen Strömungsquerschnitt verkleinert wird, um den Flusswiderstand zu vergrößern und so einen Gegendruck in der Flüssigkeitskammer der Pumpe zu erzeugen.

In einer bevorzugten Ausgestaltung der Erfindung ist vorgesehen, dass während der Druckphase der Pumpe der reduzierte Strömungsquerschnitt im Ventil fortlaufend vergrößert wird, um bei einem abnehmenden Flüssigkeitsdruck in der Pumpe eine konstante Strömungsrate aus dem Fördermodul zu gewährleisten. Weiterhin ist es denkbar, dass während der Druckphase der Pumpe das pneumatische Volumen in der Regelungskammer der Pumpe konstant gehalten wird.

In einer weiteren Ausgestaltung des Verfahrens ist vorgesehen, dass der reduzierte Strömungsquerschnitt im Ventil und der Flüssigkeitsdruck in der Pumpe während der Druckphase der Pumpe konstant gehalten werden, um eine konstante Strömungsrate aus dem Fördermodul zu gewährleisten
Weitere Einzelheiten und Vorteile der Erfindung ergeben sich aus den anhand der Figuren beschriebenen Ausführungsbeispielen. In den Figuren zeigen:
- Figur 1:: eine schematische Darstellung eines Fördermoduls eines erfindungsgemäßen Blutbehandlungsgerätes; und
- Figur 2:: einen Querschnitt durch eine Ausführungsform eines Fördermoduls eines erfindungsgemäßen Blutbehandlungsgerätes.

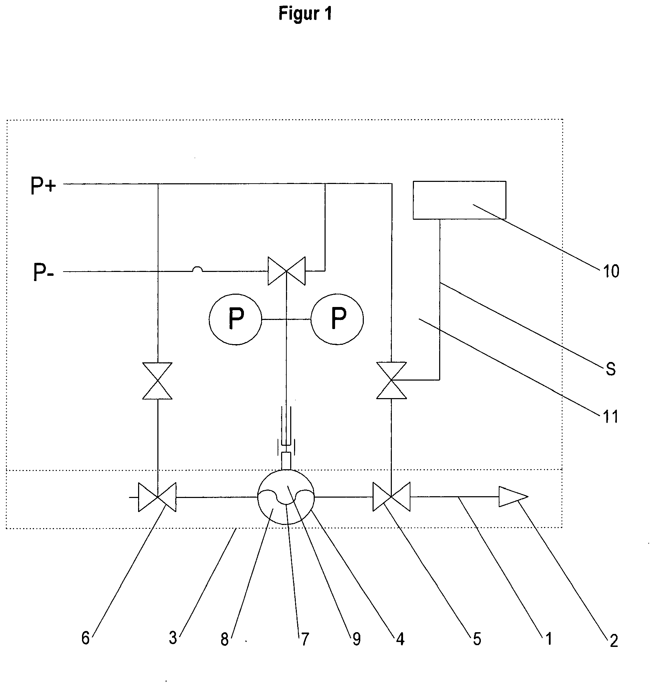

In Figur 1 ist schematisch ein Fördermodul eines erfindungsgemäßen Blutbehandlungsgerätes dargestellt, das an einer Dosierleitung 1 eines Blutbehandlungsgeräts angeordnet ist. Im gezeigten Ausführungsbeispiel handelt es sich um eine Dosierleitung 1 für ein Antikoagulationsmittel eines Dialysegerätes, welche saugseitig an ein Reservoir für eine Antikoagulationsmittellösung und druckseitig an den extrakorporalen Blutkreislauf des Gerätes angeschlossen ist. Das Fördermodul ist mit dem Bezugszeichen 3 gekennzeichnet, die Flussrichtung der Antikoagulationsmittellösung in der Dosierleitung 1 mit dem Bezugszeichen 2.

Das Fördermodul 3 umfasst eine Membranpumpe 4, ein druckseitig angeordnetes Ventil 5 und ein saugseitig angeordnetes Ventil 6. Die Ventile 5 und 6 sind identisch und können beide sowohl den Flüssigkeitsstrom unterbrechen (also als Sperrventil wirken) als auch den Strömungsquerschnitt verringern (also als Drosselventil wirken). Vorzugsweise ist vorgesehen, dass die Ventile 5 und 6 den Strömungsquerschnitt der Dosierleitung 1 stufenlos verringern können.

Die Membranpumpe 4 ist pneumatisch und umfasst eine Pumpenmembran 7, die zwischen einer Flüssigkeitskammer 8 für die zu fördernde Flüssigkeit 8 und einer pneumatischen Regelungskammer 9 angeordnet ist. In der Regelungskammer 9 herrscht im Betrieb der Pumpe ein pneumatischer Regelungsdruck P_{PNEU} und in der Flüssigkeitskammer 8 herrscht im Betrieb ein Flüssigkeitsdruck P_{HYD}. Wie eingangs erklärt wurde, geht auch von der Membran 7 ein Membrandruck P_{MEM} aus, der das im Idealfall bestehende Gleichgewicht zwischen dem Regelungsdruck P_{PNEU} und dem Flüssigkeitsdruck P_{HYD} stört.

Die Membranpumpe 4 und die beiden Ventile 5 und 6 werden durch ein Pneumatikmodul 11 angesteuert, welches mit einer Steuereinheit 10 in Verbindung steht und das in Figur 1 durch den hell hinterlegten Bereich oberhalb des Fördermoduls 3 gezeigt ist. Nicht dargestellte Fluss- und Drucksensoren in den Pneumatikleitungen und der Flüssigkeitsleitung 1 stehen ebenfalls mit der Steuereinheit in Verbindung und liefern Daten, die für die Regelung des Fördermoduls 3 bedeutsam sind.

Das Bezugszeichen S kennzeichnet eine Steuerleitung von der Steuereinheit 10 zu einem Ventil des Pneumatikmoduls 11, das seinerseits das Ventil 5 ansteuert.

Auf der Steuereinheit ist ein Algorithmus hinterlegt, der so ausgebildet ist, dass der Strömungsquerschnitt für die zu fördernde Flüssigkeit im druckseitigen Ventil 5 während einer Druckphase der Pumpe 4 (also bei geschlossenem saugseitigen Ventil 6 und Druckerhöhung in der Regelungskammer 9) gegenüber dem maximalen Strömungsquerschnitt verkleinert ist. So wird der Flusswiderstand am druckseitigen Ventil 5 vergrößert und ein Gegendruck in der Flüssigkeitskammer 8 der Membranpumpe 4 erzeugt. Dieser Offset für den Flüssigkeitsdruck in der Flüssigkeitskammer 8 (und folglich den Regelungsdruck in der Regelungskammer 9) verringert die Bedeutung des durch diese Maßnahme unverändert bleibenden Membrandrucks, wodurch die Proportionalität des Flüssigkeitsdruckverlaufs und des Regelungsdruckverlaufs verbessert wird.

Um unter diesen Voraussetzungen einen konstanten Fluss auf der Druckseite des Fördermoduls 3 zu erzeugen, kann vorgesehen sein, dass der in der Steuereinheit 10 hinterlegte Algorithmus so ausgebildet ist, dass das pneumatische Volumen in der Regelungskammer 9 der Membranpumpe 4 während der Druckphase der Pumpe konstant gehalten wird. Dadurch verringern sich aufgrund der aus der Flüssigkeitskammer 8 ablaufenden Flüssigkeit nach und nach der Flüssigkeitsdruck in der Flüssigkeitskammer 8 und korrespondierend dazu der Regelungsdruck in der Regelungskammer 9. Um dabei die Flussrate der Flüssigkeit auf der Druckseite des Fördermoduls 3 konstant halten zu können, ist der in der Steuereinheit 10 hinterlegte Algorithmus so ausgebildet, dass der Strömungsquerschnitt im druckseitigen Ventil 5 mit der Abnahme des Flüssigkeitsdrucks in der Flüssigkeitskammer 8 fortlaufend vergrößert wird.

Alternativ dazu kann vorgesehen sein, dass der in der Steuereinheit 10 hinterlegte Algorithmus so ausgebildet ist, dass der Strömungsquerschnitt im druckseitigen Ventil 5 während der Druckphase der Pumpe konstant gehalten wird, sodass ein konstanter Offset-Druck in der Flüssigkeitskammer 8 erzeugt wird. Um dabei die Flussrate der Flüssigkeit auf der Druckseite des Fördermoduls 3 konstant halten zu können, wird der Regelungsdruck während der Druckphase der Pumpe 4 durch eine fortwährende Vergrößerung des Pneumatikvolumens in der Regelungskammer 9 konstant gehalten.

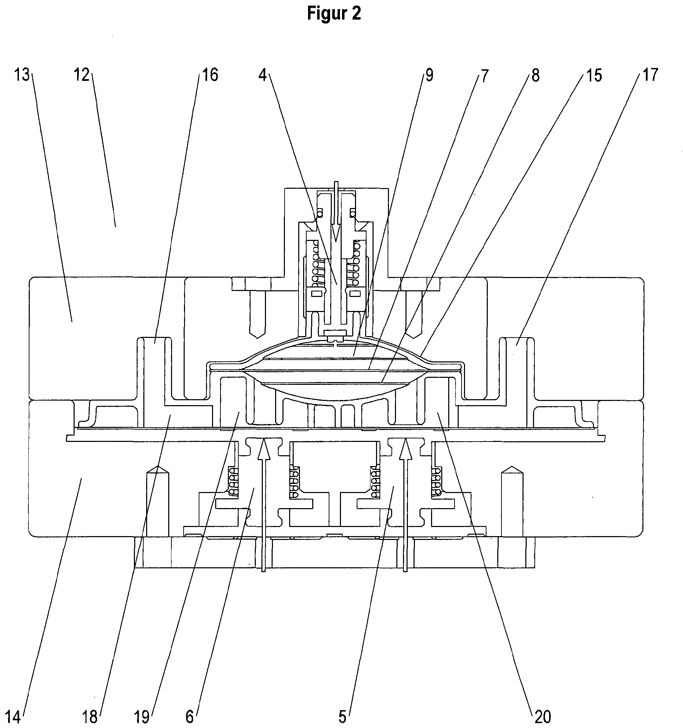

Figur 2 zeigt einen Querschnitt durch ein Fördermodul 3 eines erfindungsgemäßen Blutbehandlungsgerätes, wobei bereits in der Figur 1 schematisch dargestellte Bauteile mit identischen Bezugszeichen gekennzeichnet sind.

Wie aus dieser Abbildung ersichtlich ist, umfasst das Fördermodul ein Gehäuse 12, welches zwei gegenüberliegende Maschinenplatten 13 und 14 sowie ein dazwischen angeordnetes, entnehmbares Disposable 15 umfasst. Die Pumpe 4 ist in einer Ausnehmung einer ersten Maschinenplatte 13 versenkt und die beiden Ventile 5 und 6 sind in Ausnehmungen in der zweiten Maschinenplatte 14 versenkt, sodass die Pumpenmechanik und die Ventilmechanik auf gegenüberliegenden Seiten des Disposables 15 angeordnet ist.

Das Disposable 15 ist mehrteilig ausgebildet und umfasst eine an der ventiltragenden Platte 14 anliegende Silikonscheibe und ein an die pumpentragende Platte 13 anliegendes Silikonformteil. Es umfasst einen saugseitigen Anschluss 16 sowie einen druckseitigen Anschluss 17, zwischen denen ein Flüssigkeitsgefäß 18 angeordnet ist. Das Flüssigkeitsgefäß 18 bildet die Ventilkammer 19 des saugseitigen Ventils 6, die Flüssigkeitskammer 8 der Membranpumpe 4 sowie die Ventilkammer 20 des druckseitigen Ventils 5 aus. Auch die Regelungskammer 9 der Membranpumpe 4 wird durch das Disposable 15 gebildet.

So ist der gesamte Flüssigkeitsweg 1 vollständig vom Disposable 15 umschlossen, wodurch eine Kontamination der maschinenseitigen Teile verhindert wird.

Zusammenfassend ergibt sich, dass in einem erfindungsgemäßen Blutbehandlungsgerät durch Erzeugung eines Offset-Drucks in der Flüssigkeitskammer 8 einer in einer Dosierleitung 1 angeordneten Membranpumpe 4 eine höhere Pumpgenauigkeit erzielt werden kann. Der Offset-Druck wird durch gezielte Ansteuerung eines druckseitig der Pumpe angeordneten und adaptiv regelbaren Sperr- und Drosselventils 5 erzeugt.

## Patentansprüche

1. Blutbehandlungsgerät mit wenigstens einer Dosierleitung (1), die in einen Flüssigkeitskreislauf mündet, wobei in der Dosierleitung (1) ein Fördermodul (3) umfassend eine Membranpumpe (4), und ein druckseitig davon angeordnetes Ventil (5) angeordnet ist, **dadurch gekennzeichnet, dass** dieses das sowohl als Sperrventil als auch als Drosselventil wirken kann, wobei ferner eine Steuereinheit (10) vorgesehen ist, welche die Pumpe (4) und das Ventil (5) mittels eines Pneumatikmoduls (11) so ansteuert, dass im Ventil (5) während einer Druckphase der Pumpe (4) der Strömungsquerschnitt gegenüber dem maximalen Strömungsquerschnitt verkleinert wird, um den Flusswiderstand zu vergrößern und so einen Gegendruck in der Flüssigkeitskammer (8) der Pumpe (4) zu erzeugen.

2. Blutbehandlungsgerät nach Anspruch 1, **dadurch gekennzeichnet, dass** es sich bei dem Blutbehandlungsgerät um ein Dialysegerät handelt.

3. Blutbehandlungsgerät nach einem der vorhergehenden Ansprüche, **dadurch gekennzeichnet, dass** es sich bei der Dosierleitung (1) um eine Dosierleitung (1) für eine Lösung eines koagulationshemmenden Mittels handelt, die in einen extrakorporalen Blutkreislauf des Blutbehandlungsgeräts mündet.

4. Blutbehandlungsgerät nach einem der vorhergehenden Ansprüche, **dadurch gekennzeichnet, dass** die Membranpumpe (4) und/oder das Ventil (5) pneumatisch betrieben sind und vorzugsweise an das gemeinsames Pneumatikmodul (11) angeschlossen sind.

5. Blutbehandlungsgerät nach einem der vorhergehenden Ansprüche, **dadurch gekennzeichnet, dass** die Membranpumpe (4) und das Ventil (5) in einem gemeinsamen Gehäuse (12) des Fördermoduls (3) angeordnet sind.

6. Blutbehandlungsgerät nach Anspruch 5, **dadurch gekennzeichnet, dass** das Gehäuse (12) wenigstens zwei lösbar aneinander befestigte Teile umfasst, wobei ein erster Teil (13, 14) die Pumpenmechanik und die Ventilmechanik umfasst und ein zweiter Teil (15) wenigstens einen Abschnitt des flüssigkeitsführenden Gefäßes der Dosierleitung definiert.

7. Blutbehandlungsgerät nach Anspruch 1, **dadurch gekennzeichnet, dass** die Steuereinheit (10) so ausgebildet ist, dass während der Druckphase der Pumpe (4) der reduzierte Strömungsquerschnitt im Ventil (5) fortlaufend vergrößert wird, um bei einem abnehmenden Flüssigkeitsdruck in der Pumpe (4) eine konstante Strömungsrate aus dem Fördermodul zu gewährleisten.

8. Blutbehandlungsgerät nach Anspruch 6, **dadurch gekennzeichnet, dass** die Steuereinheit (10) so ausgebildet ist, dass während der Druckphase der Pumpe (4) das pneumatische Volumen in der Regelungskammer (9) der Pumpe (4) konstant gehalten wird.

9. Blutbehandlungsgerät nach Anspruch 1, **dadurch gekennzeichnet, dass** die Steuereinheit (10) so ausgebildet ist, dass der reduzierte Strömungsquerschnitt im Ventil (5) und der Flüssigkeitsdruck in der Pumpe (4) während der Druckphase der Pumpe (4) konstant gehalten werden, um eine konstante Strömungsrate aus dem Fördermodul (3) zu gewährleisten.

10. Verfahren zur Dosierung eines Mediums, vorzugsweise in einem Blutbehandlungsgerät gemäß einem der Ansprüche 1 bis 8, wobei zur Dosierung ein in einer Dosierleitung (1) befindliches Fördermodul (3) eingesetzt wird, das eine Membranpumpe (4), und ein druckseitig davon angeordnetes Ventil (5) umfasst, **dadurch gekennzeichnet, dass** dieses Ventil (5) sowohl als Sperrventil als auch als Drosselventil wirken kann, wobei die Pumpe (4) und das Ventil (5) mittels eines Pneumatikmoduls (11) so angesteuert werden, dass im Ventil (5) während einer Druckphase der Pumpe (4) der Strömungsquerschnitt gegenüber dem maximalen Strömungsquerschnitt verkleinert wird, um den Flusswiderstand zu vergrößern und so einen Gegendruck in der Flüssigkeitskammer (8) der Pumpe (4) zu erzeugen.

11. Verfahren nach Anspruch 10, **dadurch gekennzeichnet, dass** während der Druckphase der Pumpe (4) der reduzierte Strömungsquerschnitt im Ventil (5) fortlaufend vergrößert wird, um bei einem abnehmenden Flüssigkeitsdruck in der Pumpe (4) eine konstante Strömungsrate aus dem Fördermodul zu gewährleisten.

12. Verfahren nach Anspruch 11, **dadurch gekennzeichnet, dass** während der Druckphase der Pumpe (4) das pneumatische Volumen in der Regelungskammer (9) der Pumpe (4) konstant gehalten wird.

13. Verfahren nach Anspruch 10, **dadurch gekennzeichnet, dass** der reduzierte Strömungsquerschnitt im Ventil (5) und der Flüssigkeitsdruck in der Pumpe (4) während der Druckphase der Pumpe (4) konstant gehalten werden, um eine konstante Strömungsrate aus dem Fördermodul (3) zu gewährleisten.

## Claims

1. A blood treatment device having at least one metering line (1) which opens into a fluid circuit, wherein a conveyor module (3) is arranged in the metering line (1) and comprises a membrane pump (4) and a valve (5) which is arranged at the pressure side thereof, **characterized in that** said valve can act both as a blocking valve and as a restricting valve, with a control unit (10) further being provided which controls the pump (4) and the valve (5) by means of a pneumatic module (11) such that the flow cross-section in the valve (5) is reduced with respect to the maximum flow cross-section during a pressure phase of the pump (4) in order to increase the flow resistance and thus to generate a counter-pressure in the fluid chamber (8) of the pump (4).

2. A blood treatment device in accordance with claim 1, **characterized in that** the blood treatment device is a dialysis device.

3. A blood treatment device in accordance with one of the preceding claims, **characterized in that** the metering line (1) is a metering line (1) for a solution of a coagulation-inhibiting agent which opens into an extracorporeal blood circuit of the blood treatment device.

4. A blood treatment device in accordance with one of the preceding claims, **characterized in that** the membrane pump (4) and/or the valve (5) is/are operated pneumatically and is/are preferably connected to the common pneumatic module (11).

5. A blood treatment device in accordance with one of the preceding claims, **characterized in that** the membrane pump (4) and the valve (5) are arranged in a common housing (12) of the conveyor module (3).

6. A blood treatment device in accordance with claim 5, **characterized in that** the housing (12) comprises at least two parts which are releasably fastened to one another, wherein a first part (13, 14) comprises the pump mechanism and the valve mechanism and a second part (15) defines at least one section of the fluid-conducting vessel of the metering line.

7. A blood treatment device in accordance with claim 1, **characterized in that** the control unit (10) is configured such that the reduced flow cross-section in the valve (5) is continuously increased during the pressure phase of the pump (4) in order to ensure a constant flow rate from the conveyor module as the fluid pressure in the pump (4) reduces.

8. A blood treatment device in accordance with claim 6, **characterized in that** the control unit (10) is configured such that the pneumatic volume in the regulation chamber (9) of the pump (4) is kept constant during the pressure phase of the pump (4).

9. A blood treatment device in accordance with claim 1, **characterized in that** the control unit (10) is configured such that the reduced flow cross-section in the valve (5) and the fluid pressure in the pump (4) are kept constant during the pressure phase of the pump (4) to ensure a constant flow rate from the conveyor module (3).

10. A method for metering a medium, preferably in a blood treatment device in accordance with one of the claims 1 to 8, wherein a conveyor module (3) located in the metering line (1) is used for metering and comprises a membrane pump (4) and a valve (5) which is arranged at the pressure side thereof, **characterized in that** this valve (5) can act both as a blocking valve and as a restricting valve, with the pump (4) and the valve (5) being controlled by means of a pneumatic module (11) such that the flow cross-section in the valve (5) is reduced with respect to the maximum flow cross-section during a pressure phase of the pump (4) in order to increase the flow resistance and thus to generate a counter-pressure in the fluid chamber (8) of the pump (4).

11. A method in accordance with claim 10, **characterized in that** the reduced flow cross-section in the valve (5) is continuously increased during the pressure phase of the pump (4) in order to ensure a constant flow rate from the conveyor module as the fluid pressure in the pump (4) reduces.

12. A method in accordance with claim 11, **characterized in that** the pneumatic volume in the regulation chamber (9) of the pump (4) is kept constant during the pressure phase of the pump (4).

13. A method in accordance with claim 10, **characterized in that** the reduced flow cross-section in the valve (5) and the fluid pressure in the pump (4) are kept constant during the pressure phase of the pump (4) to ensure a constant flow rate from the conveyor module (3).

## Revendications

1. Appareil de traitement du sang doté d'au moins une conduite de dosage (1), qui débouche dans un circuit de liquide, un module de refoulement (3) comprenant une pompe à membrane (4) et une vanne (5) disposée côté refoulement de celle-ci étant disposé dans la conduite de dosage (1), **caractérisé en ce que** cette vanne peut faire office aussi bien de vanne d'arrêt que de vanne d'étranglement, une unité de commande (10) étant en outre prévue, qui commande la pompe (4) et la vanne (5) au moyen d'un module pneumatique (11) de telle manière que, dans la vanne (5), pendant une phase de compression de la pompe (4), la section de passage est réduite par rapport à la section de passage maximale, pour augmenter la résistance au flux et générer ainsi une contre-pression dans la chambre à liquide (8) de la pompe (4).

2. Appareil de traitement du sang selon la revendication 1, **caractérisé en ce que** l'appareil de traitement du sang est un appareil de dialyse.

3. Appareil de traitement du sang selon l'une des revendications précédentes, **caractérisé en ce que** la conduite de dosage (1) est une conduite de dosage (1) pour une solution d'un anticoagulant, qui débouche dans un circuit sanguin extracorporel de l'appareil de traitement du sang.

4. Appareil de traitement du sang selon l'une des revendications précédentes, **caractérisé en ce que** la pompe à membrane (4) et/ou la vanne (5) sont à actionnement pneumatique et sont de préférence raccordées au module pneumatique (11) commun.

5. Appareil de traitement du sang selon l'une des revendications précédentes, **caractérisé en ce que** la pompe à membrane (4) et la vanne (5) sont disposées dans un boîtier (12) commun du module de refoulement (3).

6. Appareil de traitement du sang selon la revendication 5, **caractérisé en ce que** le boîtier (12) comprend au moins deux parties fixées l'une à l'autre de manière détachable, une première partie (13, 14) comprenant le mécanisme de pompe et le mécanisme de vanne et une seconde partie (15) définissant au moins une portion du récipient de transfert de liquide de la conduite de dosage.

7. Appareil de traitement du sang selon la revendication 1, **caractérisé en ce que** l'unité de commande (10) est conçue de telle sorte que, pendant la phase de compression de la pompe (4), la section de passage réduite dans la vanne (5) est agrandie en continu, pour assurer un débit constant à la sortie du module de refoulement lorsque la pression de liquide dans la pompe (4) diminue.

8. Appareil de traitement du sang selon la revendication 6, **caractérisé en ce que** l'unité de commande (10) est conçue de telle sorte que, pendant la phase de compression de la pompe (4), le volume pneumatique dans la chambre de régulation (9) de la pompe (4) est maintenu constant.

9. Appareil de traitement du sang selon la revendication 1, **caractérisé en ce que** l'unité de commande (10) est conçue de telle sorte que la section de passage réduite dans la vanne (5) et la pression de liquide dans la pompe (4) sont maintenues constantes pendant la phase de compression de la pompe (4) pour assurer un débit constant à la sortie du module de refoulement (3).

10. Procédé de dosage d'un milieu, de préférence dans un appareil de traitement du sang selon l'une des revendications 1 à 8, un module de refoulement (3) situé dans une conduite de dosage (1) étant utilisé pour le dosage, ledit module de refoulement comprenant une pompe à membrane (4) et une vanne (5) disposée côté refoulement de celle-ci, **caractérisé en ce que** cette vanne (5) peut faire office aussi bien de vanne d'arrêt que de vanne d'étranglement, la pompe (4) et la vanne (5) étant commandées au moyen d'un module pneumatique (11) de telle manière que, dans la vanne (5), pendant une phase de compression de la pompe (4), la section de passage est réduite par rapport à la section de passage maximale, pour augmenter la résistance au flux et générer ainsi une contre-pression dans la chambre à liquide (8) de la pompe (4).

11. Procédé selon la revendication 10, **caractérisé en ce que**, pendant la phase de compression de la pompe (4), la section de passage réduite dans la vanne (5) est agrandie en continu, pour assurer un débit constant à la sortie du module de refoulement lorsque la pression de liquide dans la pompe (4) diminue.

12. Procédé selon la revendication 11, **caractérisé en ce que**, pendant la phase de compression de la pompe (4), le volume pneumatique dans la chambre de régulation (9) de la pompe (4) est maintenu constant.

13. Procédé selon la revendication 10, **caractérisé en ce que** la section de passage réduite dans la vanne (5) et la pression de liquide dans la pompe (4) sont maintenues constantes pendant la phase de compression de la pompe (4) pour assurer un débit constant à la sortie du module de refoulement (3).
